# EUROPÄISCHE PATENTSCHRIFT

(11) **EP 0 543 178 B1**
(45) Veröffentlichungstag und Bekanntmachung des Hinweises auf die Patenterteilung: **14.01.1998**
(21) Anmeldenummer: 92118227.5
(22) Anmeldetag: 24.10.1992
(51) Int. Cl.: C12N 9/74, A61K 38/48, A61L 25/00

(54) **Verfahren zur Herstellung eines virussicheren Thrombinkonzentrates**
Process of preparation of a virus-free thrombin concentrate
Procédé de préparation d'un concentrat de thrombine exempt de virus

(30) Priorität: 19.11.1991 DE 4137996
(43) Veröffentlichungstag der Anmeldung: 26.05.1993
(73) Patentinhaber: Centeon Pharma GmbH, 35002 Marburg (DE)
(72) Erfinder: Karges, Hermann E., W-3550 Marburg (DE); Naumann, Horst, W-3550 Marburg (DE)

(56) Entgegenhaltungen:
- EP-A- 0 378 798
- EP-A- 0 443 724
- DE-A- 3 229 132
- US-A- 2 394 566
- US-A- 4 357 321
- THE JOURNAL OF BIOLOGICAL CHEMISTRY, Bd. 246, Nr. 19, 1971, BALTIMORE, USA, Seiten 6106-6114; MANN ET AL: 'MULTIPLE ACTIVE FORMS OF THROMBIN II.MECHANISM OF PRODUCTION FROM PROTHROMBIN'
- CHEMICAL ABSTRACTS, vol. 66, no. 19, 8. Mai 1967, Columbus, Ohio, US; abstract no. 83733c; SHAPIRO ET AL: 'THE PURIFICATION OF HUMAN PROTHROMBIN' Seite 7829 ;Spalte 1 ; & THROMB.DIATH.HAEMORRH., Bd. 16, Nr. 3-4, 1966, Seiten 469-490
- Blood, 5, 1950, Seiten 421-423
- Nature, 195, 1962, no. 4847, Seite 1305

## Beschreibung

Die Erfindung betrifft ein Verfahren, das in einfacher Weise aus einer pasteurisierten Lösung eines Prothrombinkomplexes eine virussichere Thrombinpräparation herzustellen erlaubt.

Zur Gewinnung von Thrombin sind mehrere Verfahren beschrieben, die von einem partiell gereinigten Prothrombin ausgehen und dieses dann durch Zusatz von Gewebethromboplastin und Ca-Ionen in Thrombin umwandeln.

Auch Methoden zur Umwandlung eines ungereinigten Prothrombinkonzentrates in Thrombin durch hohe Salzkonzentrationen sind bekannt. Diese Umwandlung erfolgt nur, wenn alle Faktoren des Prothrombinkomplexes in ausreichender Menge im Gemisch vorhanden sind. Ein über einen Anionenaustauscher gereinigter Prothrombinkomplex läßt sich mit Salz aktivieren, wenn Autoprothrombin C (F X) zugesetzt wird. Auch die Anwesenheit von F VII ist wesentlich.

Bedingt durch die Gefahr mit Proteinen menschlichen oder tierischen Ursprungs Krankheitserreger viralen Ursprungs (z. B. Hepatitis, AIDS, BSE) zu übertragen, sind bei der Herstellung von Konzentraten, die solche Proteine enthalten, Inaktivierungsmaßnahmen für Pathogene erforderlich.

Für die Herstellung von Thrombin sind eine Reihe von Verfahren bekannt, die einen Inaktivierungsschritt für Pathogene enthalten, beispielsweise durch trockene Erhitzung.

Es sind auch Verfahren zur Virusinaktivierung in wässrigen Thrombinlösungen bekannt (DE 38 09 991).

EP 0 378 798, entsprechend DE 38 43 126, beschreibt ein Verfahren, bei dem der Prothrombinkomplex an einen Anionenaustauscher gebunden und mit Ca-Ionen, Gewebethromboplastin oder aktiviertem F Xa aktiviert wird.

Alle Verfahren, die zur Aktivierung des Prothrombinkomplexes Gewebethromboplastin benutzen, haben den Nachteil, daß dieses anschließend nicht entfernt werden kann und eine Quelle der Verunreinigung des Produktes darstellt.

Aktivierungen mit hohen Konzentrationen von Salzen, die Ca-Ionen komplexieren, wie Natrium-Citrat, haben den Vorteil, daß der Prothrombinkomplex nicht zusätzlich durch Gewebeproteine kontaminiert wird. Allerdings läßt sich ein über DEAE-Austauscher gereinigtes Prothrombinkonzentrat nicht zu Thrombin aktivieren, wenn man nicht aktivierten F X oder Gewebethromboplastin zusetzt. Es wäre auch vorteilhaft, die Virusinaktivierung bereits im Prothrombinkomplex durchzuführen, damit das im Vergleich zum Prothrombin labile Enzym Thrombin nicht den rauhen Methoden der Virusinaktivierung ausgesetzt werden muß und durch Strukturumwandlungen an Nativität verliert.

Es wurde nun überraschenderweise gefunden, daß ein über Anionenaustauscher gereinigter und pasteurisierter Prothrombinkomplex durch Zugabe eines löslichen Salzes mit einem Anion, das mit Calcium ein schwerlösliches Salz oder einen löslichen Komplex bildet, in einer Konzentration von mindestens 0.5 mol/l zu Thrombin aktiviert werden kann, wenn der Ansatz eine katalytische Menge Thrombin enthält (Tab. 1, Spalte b). Ohne den Salzzusatz bewirkt die gleiche Thrombin-Menge nur eine ungenügende Aktivierung des Prothrombins (Tab. 2, Spalte c). Weiterhin ist die Aktivierung temperaturabhängig.

Gegenstand der Erfindung ist deshalb ein Verfahren zur Herstellung einer gereinigten und virussicheren Thrombin-Präparation, dadurch gekennzeichnet, daß einer Lösung eines über einen Anionenaustauscher gereinigten und einer Virus-Inaktivierung unterworfenen Prothrombin-Komplexes ein lösliches Salz mit einem Anion, das mit Calcium ein schwerlösliches Salz oder einen löslichen Komplex bildet, in einer Konzentration von mindestens 0.5 mol/l zugegeben und die Lösung mit einer katalytischen Menge Thrombin behandelt wird.

Die katalytische Menge Thrombin kann während des Reinigungsverfahrens entstanden sein. Andernfalls wird Thrombin zugesetzt.

Eine katalytische Menge Thrombin soll Thrombin in einer Konzentration von größer als Null bis 200, vorzugsweise 10 bis 50 Einheiten pro ml bedeuten.

Der Prothrombin-Komplex kann aus tierischem Plasma gewonnen sein.

Vorzugsweise wird ein über DEAE-Ionenaustauscher gereinigter Prothrombinkomplex, der beispielsweise nach EP 0 137 428 10 h bei 60°C pasteurisiert wird, verwendet.

Anstelle einer Pasteurisierung können die Viren jedoch auch auf eine beliebige andere Weise inaktiviert werden.

Als calciumbindendes Anion wird vorzugsweise das Sulfat-, Citrat-, Phosphat- oder Oxalat-Anion, besonders Citratanion, verwendet. Das entsprechende Salz, vorzugsweise ein Alkali- oder Ammoniumsalz wird in einer Konzentration von 0.5 mol/l bis zur jeweiligen Sättigungsgrenze verwendet.

Weitere bevorzugte Ausführungsformen sind dadurch gekennzeichnet, daß Prothrombinkomplex aus tierischem Plasma eingesetzt wird oder daß bei 0°C bis 50°C, vorzugsweise bei 28°C, mit Thrombin 2 - 100 Stunden, vorzugsweise 5 - 20 Stunden, behandelt wird.

Mit dem erfindungsgemäßen Verfahren läßt sich auf einfache Weise ein natives, hochreines und virussicheres Thrombinkonzentrat herstellen, das als Haemostyptikum oder in einem Gewebekleber auf der Basis von Fibrinogen verwendet werden kann.

### Beispiele

### Beispiel 1

16 ml pasteurisiertes Human-Prothrombinkonzentrat mit 65 E F II/ml wurden mit 20 E/ml Human-Thrombin und 4 g (25 % w/v) Tri-Natrium-Citrat versetzt und nach Einlösen des Salzes bei 28°C inkubiert. Nach verschiedenen Zeiten wurde die erhaltene Thrombinaktivität bestimmt (Tabelle 1, Spalte b), am Ende der Aktivierung das Citrat ausdialysiert und das Thrombin nach Stabilisierung und Einstellung der gewünschten Aktivität lyophilisiert.

**Tabelle 1**

| Aktivierung von an Anionen-Austauschern gereinigten Prothrombinkomplex mit gesättigter Citratlösung in Abhängigkeit von der Temperatur | | | |
|---|---|---|---|
| | Thrombinaktivität (IE/ml) der Ansätze | | |
| Zeit (Std) | a) bei 4°C | b) bei 28°C | c) bei 37°C |
| 0 | 15 | 20 | 20 |
| 1 | n.b. | 61 | 63 |
| 3 | 13 | 350 | 1084 |
| 5 | n.b. | 1695 | 3351 |
| 10 | 18 | 5858 | 5141 |
| 21 | n.b. | 8492 | 7393 |
| 25 | 14 | 8978 | 6991 |
| 45 | 94 | 8109 | 7575 |
| 70 | 6800 | n.b.* | n.b. |
| 96 | 7700 | n.b. | n.b. |
| Höchste Thrombinaktivität pro 1 IE F II | | 138 | 117 |

| | | | |
|---|---|---|---|
| *n.b. = nicht bestimmt | | | |

### Beispiel 2

18 ml pasteurisiertes Human-Prothrombinkonzentrat mit 65 E F II/ml wurden mit 20 E/ml Human-Thrombin und 4,5 g Tri-Natrium-Citrat versetzt und nach Einlösen des Salzes bei 37°C inkubiert. Nach verschiedenen Zeiten wurde die erhaltene Thrombinaktivität bestimmt (Tabelle 1).

### Beispiel 3

8 ml pasteurisiertes Human-Prothrombinkonzentrat mit 70 E F II/ml wurden mit 20 E/ml Humanthrombin/ml versetzt und ohne Zusatz eines Salzes mit calciumbindenden Anion bei 37°C inkubiert. Die nach verschiedenen Zeiten erhaltenen Thrombinaktivitäten sind in der Tabelle 2, Spalte c dargestellt.

### Beispiel 4

10 ml pasteurisiertes Human-Prothrombinkonzentrat mit 80 E F II/ml wurde ohne Zusatz von Thrombin mit 2,5 g Tri-Natrium-Citrat versetzt und nach dem Einlösen des Salzes bei 28°C inkubiert. Nach verschiedenen Zeiten wurden Proben entnommen und Thrombin-Aktivität bestimmt (Tabelle 2, Spalte b).

**Tabelle 2**

| Aktivierung von an Anionen-Austauschern gereinigten Prothrombinkomplex in Abhängigkeit von der Spezies des Prothrombins, von Thrombin- und Zusatz eines Salzes mit Ca-bindendem Anion | | | |
|---|---|---|---|
| | Thrombinaktivität (IE/ml) der Ansätze | | |
| Zeit (Std) | a) bov.Prothr., 28°C,Citr. | b) Citr.,ohne Thrombin, 28°C | c)Thrombin, ohne Salzzusatz, 37°C |
| 0 | 11 | < 0,1 | 20 |
| 2 | 224 | n.b.* | 36 |
| 4 | 767 | < 0,1 | 52 |
| 6 | 2833 | n.b. | 93 |
| 11 | 4718 | < 0,1 | 222 |
| 25 | 5870 | < 0,1 | 846 |
| 45 | 5921 | < 0,1 | 1090 |
| 70 | n.b. | < 0,1 | 1409 |
| 101 | n.b. | n.b. | 1511 |
| Höchste Thrombinaktivität pro 1 IE F II 118 | | | 22 |

| | | | |
|---|---|---|---|
| n.b.* = nicht bestimmt | | | |

### Beispiel 5

20 ml pasteurisiertes Prothrombinkonzentrat aus Rinderplasma mit 50 E F II/ml wurde mit 10 E/ml Rinderthrombin und 5 g Tri-Natrium-Citrat versetzt und bei 28°C inkubiert. Nach verschiedenen Zeiten wurde die erhaltene Thrombinaktivität bestimmt (Tabelle 2, Spalte a).

### Beispiel 6

500 ml virusinaktiviertes Human-Prothrombinkonzentrat mit 75 E F II/ml wurde mit 15 E/ml Thrombin und 125 g Tri-Natrium-Citrat versetzt. Nach Einlösen des Salzes bei Raumtemperatur wurde die Lösung bei 4°C inkubiert. Nach verschiedenen Zeiten wurden Proben entnommen und die Thrombinaktivität bestimmt (Tabelle 1, Spalte a).

## Patentansprüche

1. Verfahren zur Herstellung einer gereinigten und virussicheren Thrombin - Präparation, dadurch gekennzeichnet, daß einer Lösung eines über einen Anionenaustauscher gereinigten und einer Virus-Inaktivierung unterworfenen Prothrombinkomplexes ein lösliches Salz mit einem Anion, das mit Calcium ein schwerlösliches Salz oder einen löslichen Komplex bildet, in einer Konzentration von mindestens 0.5 mol/l zugegeben und die Lösung mit einer katalytischen Menge Thrombin behandelt wird.

2. Verfahren nach Anspruch 1, dadurch gekennzeichnet, daß Thrombin in einer Konzentration von größer als Null bis 200, vorzugsweise 10 bis 50 Einheiten pro ml verwendet wird.

3. Verfahren nach Anspruch 1, dadurch gekennzeichnet, daß Prothrombinkomplex aus tierischem Plasma verwendet wird.

4. Verfahren nach Anspruch 1, dadurch gekennzeichnet, daß Prothrombinkomplex aus menschlichem Plasma verwendet wird.

5. Verfahren nach Anspruch 1, dadurch gekennzeichnet, daß bei 0°C bis 50°C, vorzugsweise bei 28°C, mit Thrombin behandelt wird.

6. Verfahren nach Anspruch 1, dadurch gekennzeichnet, daß ein über DEAE-Ionenaustauscher gereinigter Prothrombinkomplex verwendet wird.

7. Verfahren nach Anspruch 1, dadurch gekennzeichnet, daß das calciumbindende Anion Sulfat-, Citrat-, Phosphat - oder Oxalat - Anion ist.

8. Verfahren nach Anspruch 1, dadurch gekennzeichnet, daß das Salz in einer Konzentration von 0.5 bis zur jeweiligen Sättigungsgrenze verwendet wird.

9. Verfahren nach Anspruch 1, dadurch gekennzeichnet, daß die katalytische Menge Thrombin während der Reinigung und/oder Virusinaktivierung des Prothrombinkomplexes erzeugt wird.

## Claims

1. A process for the production of a purified and virus-free preparation of thrombin, which comprises a soluble salt which has an anion that forms a sparingly-soluble salt or a soluble complex with calcium being added in a concentration of at least 0.5 mol/l to a solution of a prothrombin complex which has been purified on an anion exchanger and subjected to virus inactivation, and the solution being treated with a catalytic quantity of thrombin.

2. The process as claimed in claim 1, wherein thrombin is used in a concentration of greater than zero up to 200, preferably 10 to 50, units per ml.

3. The process as claimed in claim 1, wherein prothrombin complex from animal plasma is used.

4. The process as claimed in claim 1, wherein prothrombin complex from human plasma is used.

5. The process as claimed in claim 1, wherein treatment with thrombin takes place at 0°C to 50°C, preferably at 28°C.

6. The process as claimed in claim 1, wherein prothrombin complex that has been purified on DEAE ion exchangers is used.

7. The process as claimed in claim 1, wherein the calcium-binding anion is sulfate, citrate, phosphate or oxalate anion.

8. The process as claimed in claim 1, wherein the salt is used in a concentration of from 0.5 up to the particular saturation limit.

9. The process as claimed in claim 1, wherein the catalytic quantity of thrombin is produced during the purification and/or subjecting to virus inactivation of the prothrombin complex.

## Revendications

1. Procédé pour la préparation d'une composition de thrombine purifiée et exempte de virus, caractérisé en ce que l'on ajoute à une solution d'un complexe de prothrombine, purifié sur un échangeur d'anions et soumis à une inactivation des virus, un sel soluble avec un anion qui forme avec le calcium un sel peu soluble ou un complexe soluble, à une concentration d'au moins 0,5 mole/l, et on traite la solution par une quantité catalytique de thrombine.

2. Procédé selon la revendication 1, caractérisé en ce que la thrombine est utilisée à une concentration de >0 à 200, de préférence de 10 à 50 unités par ml.

3. Procédé selon la revendication 1, caractérisé en ce que l'on utilise un complexe de prothrombine provenant de plasma animal.

4. Procédé selon la revendication 1, caractérisé en ce que l'on utilise un complexe de prothrombine provenant de plasma humain.

5. Procédé selon la revendication 1, caractérisé en ce que l'on effectue le traitement par la thrombine à 0-50°C, de préférence à 28°C.

6. Procédé selon la revendication 1, caractérisé en ce que l'on utilise un complexe de prothrombine purifié sur échangeur d'ions-DEAE.

7. Procédé selon la revendication 1, caractérisé en ce que l'anion fixant le calcium est l'anion sulfate, citrate, phosphate ou oxalate.

8. Procédé selon la revendication 1, caractérisé en ce que le sel est utilisé à une concentration allant de 0,5 à la limite de saturation respective.

9. Procédé selon la revendication 1, caractérisé en ce que la quantité catalytique de thrombine est engendrée pendant la purification et/ou l'inactivation des virus du complexe de prothrombine.
